(19) Europäisches Patentamt
European Patent Office
Office européen des brevets

(11) Veröffentlichungsnummer: **0 153 561**
**B1**

(12) # EUROPÄISCHE PATENTSCHRIFT

(45) Veröffentlichungstag der Patentschrift: **12.08.87**

(51) Int. Cl.⁴: **C 07 C 119/045,** C 08 G 18/75

(21) Anmeldenummer: **85100290.7**

(22) Anmeldetag: **14.01.85**

(54) **Neue Diisocyanate und ihre Verwendung zur Herstellung von Polyurethankunststoffen.**

(30) Priorität: **26.01.84 DE 3402623**

(43) Veröffentlichungstag der Anmeldung: **04.09.85 Patentblatt 85/36**

(45) Bekanntmachung des Hinweises auf die Patenterteilung: **12.08.87 Patentblatt 87/33**

(84) Benannte Vertragsstaaten: **DE FR GB IT**

(56) Entgegenhaltungen:
**AT-B-253 222**
**DE-A-2 353 151**
**DE-A-3 135 948**
**DE-B-1 022 222**
**US-A-3 799 965**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Klein, Gerhard, Dr., von- Flotow- Strasse 7, D-4019 Monheim (DE)**
Erfinder: **Arlt, Dieter, Dr., Rybniker Strasse 2, D-5000 Köln 80 (DE)**

**EP 0 153 561 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue aliphatisch-cycloaliphatische Diisocyanate, die neben einer sterisch ungehinderten, aliphatisch gebundenen Isocyanatgruppe eine sterisch gehinderte, cycloaliphatisch gebundene Isocyanatgruppe aufweisen und die Verwendung der neuen Diisocyanate als Ausgangsmaterial bei der Herstellung von Polyurethankunststoffen.

Die in der technischen Polyurethanchemie eingesetzten Diisocyanate mit aliphatisch und/oder cycloaliphatisch gebundenen Isocyanatgruppen wie z. B. 1-Isocyanato-3,3,5-trimethyl-5-isocyanatomethyl-cyclohexan (Isophorondiisocyanat), 4,4'-Diisocyanato-dicyclohexylmethan oder Hexamethylendiisocyanat eignen sich wegen ihres aliphatischen Charakters hervorragend zur Herstellung von lichtechten Polyurethankunststoffen. Diese Diisocyanate werden daher insbesondere zur Herstellung von Polyurethanlacken oder zur Herstellung von Lackpolyisocyanaten eingesetzt. Ein Nachteil dieser wichtigen aliphatischen und/oder cycloaliphatischen Diisocyanate des Standes der Technik ist in dem Umstand zu sehen, daß ihre Isocyanatgruppen eine gleiche oder ähnliche Reaktivität gegenüber Verbindungen mit Isocyanatreaktiven Gruppen aufweisen, was z. B. Umsetzungen erschwert, bei denen nur eine Isocyanatgruppe zur Reaktion gebracht werden soll, z. B. die Herstellung von NCO-Präpolymeren durch Reaktion einer Isocyanatgruppe des Diisocyanats mit Hydroxylgruppen von Polyhydroxylverbindungen der aus der Polyurethanchemie bekannten Art.

Es war daher die der Erfindung zugrundeliegende Aufgabe, neue alipahtisch-cycloaliphatische Diisocyanate zur Verfügung zu stellen, deren Isocyanatgruppen sich bezüglich ihrer Reaktivität so deutlich voneinander unterscheiden, daß die Herstellung von, freie Isocyanatgruppen aufweisenden, NCO-Präpolymeren ohne nennenswerte Kettenverlängerungsreaktion ermöglicht wird.

Diese Aufgabe konnte durch die Bereitstellung der neuen erfindungsgemäßen Diisocyanate gelöst werden.

Die AT-B-253 222 beschreibt zwar bereits ein aliphatisches Diisocyanat, nämlich das 1-Isocyanato-3,3,5-trimethyl-5-isocyanatomethyl-cyclohexan(IPDI), welches ebenfalls Isocyanatgruppen einer unterschiedlichen Reaktivität aufweist. Hierunter ist jedoch, wie aus Untersuchungen des Anmelders hervorgeht, ein Reaktivitätsverhältnis von ca. 4,5:1 zu verstehen. Bei den nachstehend näher beschriebenen erfindungsgemäßen Diisocyanaten liegt dieses Reaktivitätsverhältnis bei ca. 75:1. Im übrigen ist der AT-B-253 222 an keiner Stelle ein Hinweis zu entnehmen, wie Diisocyanate mit primär bzw. tertiär gebundenen Isocyanatgruppen der Art der nachstehend näher beschriebenen erfindungsgemäßen Diisocyanate zugänglich sein könnten.

Die US-A-3 799 965 beschreibt zwar Diisocyanate mit primär bzw. tertiär gebundenen Isocyanatgruppen, jedoch unterscheiden sich die Diisocyanate dieser Vorveröffentlichung sowohl bezüglich der Art ihrer Herstellung als auch bezüglich ihrer Konstitution ganz wesentlich von den erfindungsgemäßen Diisocyanaten. Die Diisocanate dieser Vorveröffentlichung weisen einerseits einen NCO-Gehalt von nur ca. 23 % auf und stellen Isomerengemische dar, in denen eine Komponente vorliegt, in der die Isocyanatgruppe und die Isocyanatoalkylgruppe ortho-ständig zueinander angeordnet sind, was bei den erfindungsgemäßen Diisocyanaten nicht der Fall sein kann. Im übrigen liegen in den Diisocyanaten der Vorveröffentlichung langkettige aliphatische Kohlenwasserstoffreste vor, die zusammen 13 Kohlenstoffatome aufweisen, ein Strukturmerkmal, welches auf die Art der Herstellung der Diisocyanate zurückzuführen ist, und welches ebenfalls einen strukturellen Unterschied gegenüber den erfindungsgemäßen Diisocyanaten begründet. Die Herstellung der Diisocyanate der Vorveröffentlichung erfolgt im übrigen nach einem grundsätzlich andersartigen Verfahren, welches keine technische Alternative zur nachstehend näher beschriebenen Herstellung der erfindungsgemäßen Diisocyanate darstellen kann, da die hierzu erforderlichen, konjugiert ungesättigten $C_{11}$-Carbonsäuren keine wohlfeilen Ausgangsmaterialien darstellen.

Gegenstand der Erfindung sind gegebenenfalls als Isomerengemische vorliegende Diisocyanate der Formel I

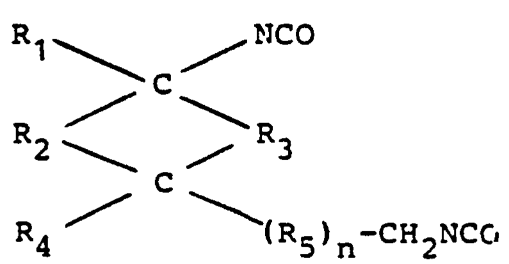

I

in welcher

$R_1$ für einen Alkylrest mit 1 bis 4 Kohlenstoffatomen steht,

$R_2$ und $R_3$ für gleiche oder verschiedene Reste stehen und jeweils lineare oder verzweigte, zweiwertige Kohlenwasserstoffreste mit 1 his 4 Kohlenstoffatomen bedeuten,

$R_4$ für Wasserstoff oder einen Alkylrest mit 1 bis 4 Kohlenstoffatomen steht,

$R_5$ für einen linearen oder verzweigten, gesättigten zweiwertigen Kohlenwasserstoffrest mit 1 bis 4 Kohlenstoffatomen steht und

n für 0 oder 1 steht.

Gegenstand der Erfindung ist auch die Verwendung der neuen Diisocyanate, gegebenenfalls in mit Blockierungsmitteln für Isocyanatgruppen blockierter Form als Isocyanatkomponente bei der Herstellung von Polyurethankunststoffen.

Zu den bevorzugten erfindungsgemäßen Diisocyanaten gehören solche der letztgenannten Formel, für welche

$R_1$ für einen Methylrest steht,

$R_2$ und $R_3$ gleiche oder verschiedene lineare, zweiwertige, gesättigte Kohlenwasserstoffreste mit 1 bis 3 Kohlenstoffatomen bedeuten, mit der Maßgabe, daß die Summe der Anzahl der Kohlenstoffatome der Reste $R_2$ und $R_3$ 3 bis 6, insbesondere 3 oder 4 beträgt,

$R_4$ für Wasserstoff oder einen Methylrest steht, und

$R_5$ und n die obengenannte Bedeutung haben.

Besonders bevorzugte erfindungsgemäße Diisocyanate sind z. B. 1-Isocyanato-1-methyl-4(3)-isocyanatomethylcyclohexan, welches im allgemeinen als Gemisch der 4- und 3-Isocyanatomethyl-Isomeren vorliegt, 1-Isocyanato-1-methyl-4-(4-isocyanatobut-2-yl)-cyclohexan oder 1-Isocyanato-1,2,2-trimethyl-3-(2-isocyanatoethyl)-cyclopentan.

Die erfindungsgemäßen Diisocyanate können beispielsweise hergestellt werden, indem man ungesättigte Amine der allgemeinen Formel II

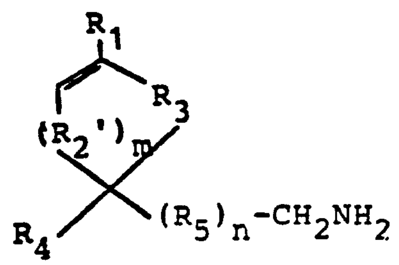

II

in welcher

$R_2'$ für einen zweiwertigen, gesättigten, linearen oder verzweigten Kohlenwasserstoffrest mit 1 bis 3 Kohlenstoffatomen steht,

m für 0 oder 1 steht und

$R_1$, $R_3$, $R_4$, $R_5$ und n die obengenannte Bedeutung haben, oder Aminoalkohole der allgemeinen Formel III,

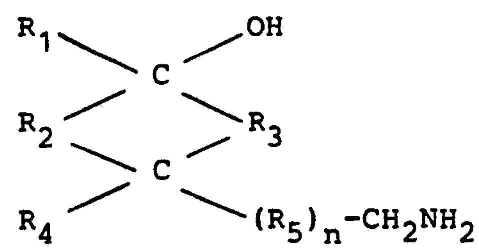

in welcher $R_1$, $R_2$, $R_3$, $R_4$, $R_5$ und n die oben angegebene Bedeutung haben, in einer Ritter-Reaktion mit Blausäure zu den Diaminen der allgemeinen Formel IV,

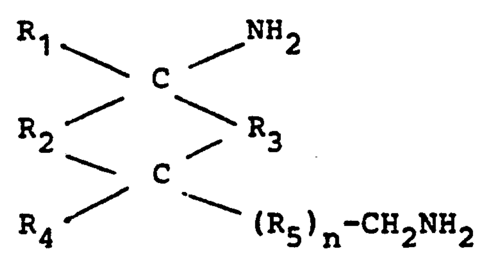

IV

umsetzt.

Aus den Diaminen der allgemeinen Formel IV erhält man durch Phosgenierung die Diisocyanate der allgemeinen Formel I.

Die ungesättigten Amine der allgemeinen Formel II sind entweder bekannt oder können aus Verbindungen der allgemeinen Formel V,

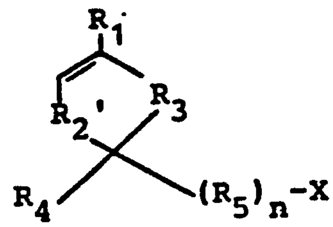

V

in welcher
X für -CHO oder -CN steht und
$R_1$ $R_2'$, $R_3$, $R_4$, $R_5$ und n die bereits obengenannte Bedeutung haben,
durch katalytische Hydrierung gewonnen werden.

Die Grundsubstanzen der allgemeinen Formel V können beispielsweise durch die bekannte Diels-Alder-Reaktion aus den entsprechenden, konjugierte Doppelbindungen aufweisenden Bisolefinen und ungesättigten Nitrilen oder Aldehyden oder durch Hydroformylierung der entsprechenden ungesättigten Kohlenwasserstoffe erhalten werden. So stellt beispielsweise das als Stellungsisomerengemisch vorliegende Diels-Alder-Adukkt der Formel VIa und VIb

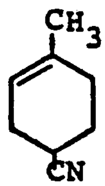

VIa

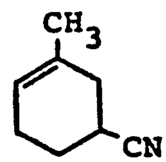

VIb

die Grundsubstanz des ebenfalls als Stellungsisomerengemisch vorliegenden 1-Isocyanato-1-methyl-4(3)-isocyanatomethylcyclohexans und die durch Hydroformylierung von Limonen erhältliche Verbindung der Formel VII

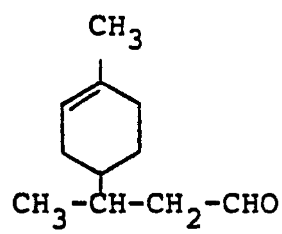

VII

die Grundsubstanz von 1-Isocyanato-1-methyl-4(4-isocyanatobut-2-yl)-cyclohexan dar. Die entsprechende Grundsubstanz von 1-Isocyanato-1,2,2-trimethyl-3-(2-isocyanatoethyl)-cyclopentan ist Campholenaldehyd der Formel VIII

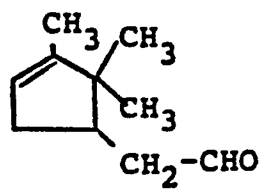

VIII

Weitere, der allgemeinen Formel V entsprechende, Grundsubstanzen können in Analogie zu diesen Ausführungen durch geeignete Auswahl der zu ihrer Herstellung benutzten Ausgangsmaterialien erhalten werden. Die Verbindungen VI-VIII sind im übrigen literaturbekannt (VI: Chem. Abstr. 71, 112475 F; VII: EP-A-0 008 459; VIII: Berichte 68B 1430 (1935)).

Die Ritter-Reaktion der ungesättigten Amine der allgemeinen Formel II oder der Aminoalkohole der allgemeinen Formel III wird in Gegenwart einer starken Säure wie Schwefelsäure, Phosphorsäure, Alkyl- oder Arylsulfonsäuren oder Trifluoressigsäure durchgeführt. Bevorzugt wird Schwefelsäure verwendet. Der Wassergehalt der Säure kann zwischen 5 und 50 % betragen, vorzugsweise jedoch 25 bis 35 %. Pro Mol ungesättigtes Amin verwendet man 1 bis 3 Mol Säure, vorzugsweise 2 Mol. Bezogen auf das ungesättigte Amin der allgemeinen Formel II oder den Aminoalkohol der allgemeinen Formel III wird eine äquimolare Menge oder

ein Überschuß von bis zu einem Mol Blausäure verwendet. In einer bevorzugten Verfahrensweise wird das ungesättigte Amin der allgemeinen Formel II zu der Säure gegeben und anschließend die Blausäure zugefügt. Die Temperatur wird während der Zugabe des Amins zwischen 0 und 25°C und während der Blausäurezugabe zwischen 10 und 50°C, vorzugsweise zwischen 30 und 45°C gehalten. Nach einer Reaktionszeit von 2 bis 10 Stunden, vorzugsweise 4 bis 6 Stunden wird das gebildete Formamid sauer hydrolysiert und das gebildete Diamin der allgemeinen Formel IV durch Neutralisation mit einer Base wie z. B. Natronlauge freigesetzt.

Das durch die Ritter-Reaktion gewonnene Diamin der allgemeinen Formel IV kann in an sich bekannter Weise phosgeniert werden. Dazu wird z. B. das Diamin in einem inerten Lösungsmittel mit Kohlendioxid bei Temperaturen zwischen 0 und 150°C, vorzugsweise zwischen 80 und 100°C gesättigt. Das entstandene Additionsprodukt wird dann bei 0 bis 200°C, vorzugsweise bei 120 bis 150°C mit Phosgen zum Diisocyanat der allgemeinen Formel I umgesetzt. Es können alle indifferenten Lösungsmittel verwendet werden, deren Siedetemperatur für die Phosgenierung hoch genug liegt und die zum Diisocyanat eine ausreichende Siedepunktdifferenz aufweisen. Bevorzugt sind Chlorbenzole, Nitrobenzole, Xylole, Tetralin und Decalin.

In einer anderen Ausführungsform der Phosgenierung wird das Diamin in einem indifferenten Lösungsmittel zu einer Lösung von Phosgen in demselben Lösungsmittel bei Temperaturen zwischen -20°C und +50°C gegeben. Der Phosgen überschuß bezogen auf das Diamin sollte 2 bis 10 Mol betragen, vorzugsweise 4 bis 6 Mol. Eine weitere Umsetzung des Additionsprodukts zum Diisocyanat erfolgt dann bei einer Temperatur von 20 bis 200°C, vorzugsweise zwischen 120°C und 150°C.

Die auf diese Weise erhaltenen erfindungsgemäßen Diisocyanate weisen einen NCO-Gehalt von 30 bis 48 Gew.-% auf und stellen im allgemeinen Gemische von Stereoisomeren dar. Darüber hinaus kann es sich bei den erfindungsgemäßen Diisocyanaten, insbesondere im Falle der Verwendung von ungesättigten Nitrilen der allgemeinen Formel V als Grundsubstanz, die durch Diels-Alder-Reaktion erhalten worden sind, um Gemische von Stellungsisomeren handeln. Die erfindungsgemäßen Diisocyanate der allgemeinen Formel I stellen wertvolle Ausgangsmaterialien zur Herstellung von Polyurethankunststoffen nach dem Isocyanat-Polyadditionsverfahren dar. Hierzu können die Diisocyanate sowohl als solche als auch in mit Blockierungsmitteln für Isocyanatgruppen wie z. B. ε-Caprolactam, Methyl-ethyl-ketoxim, Malonsäurediethylester oder Acetessigsäureethylester blockierter Form zum Einsatz gelangen. Die neuen Diisocyanate eignen sich insbesondere zur Herstellung von Polyurethankunststoffen nach dem Zweistufen-Princip, wobei in einer ersten Stufe zunächst aus den erfindungsgemäßen Diisocyanaten und unterschüssigen Mengen an organischen Polyhydroxylverbindungen der in der Polyurethanchemie an sich bekannten Art freie Isocyanatgruppen aufweisende Präpolymere hergestellt werden, die anschließend in einer zweiten Reaktionsstufe mit geeigneten Kettenverlängerungsmitteln in das hochmolekulare Polyurethan überführt werden. Alle genannten Verfahren zur Herstellung von Polyurethankunststoffen unter Verwendung von erfindungsgemäßen Diisocyanate finden insbesondere auf dem Lacksektor, d.h. zur Herstellung von Ein- und Zweikomponenten-Polyurethanlacken Verwendung.

Die in den nachfolgenden Beispielen gemachten Prozentangaben beziehen sich auf Gewichtsprozente. Alle Druckangaben bei den Siedepunkten sind in mbar.

**Beispiele**

In den nachfolgenden Beispielen werden die folgenden ungesättigten Amine eingesetzt:

**Ungesättiges Amin A:**

605 g 4(5)-Cyano-1-methylcyclohexen werden in einem Rührautoklaven in 500 ml flüssigem Ammoniak gelöst und bei 90°C und einem Wasserstoffdruck von 100 bar über 40 g Raney-Nickel-Eisen hydriert. Nach dem Abdampfen des Ammoniaks wird vom Katalysator abfiltriert und i. Vak. destilliert. Man erhält 550 g (88 %) 4(5)-Aminomethyl-1-methylcyclohexen, $Kp._{10}$ 78 - 80°C. Das Gewichtsverhältnis der 4- und 5-Isomeren liegt bei ca. 80:20.

**Ungesättigtes Amin B:**

830 g 3-[1-Methylcyclohexen-4-yl]-butyraldehyd werden in einem Rührautoklaven in 800 ml flüssigem Ammoniak gelöst und bei 90°C und einem Wasserstoffdruck von 100 bar über 50 g Raney-Nickel-Eisen hydriert. Nach dem Abdampfen des Lösungsmittels wird vom Katalysator abfiltriert und i. Vak. destilliert. Man erhält 720 g (86 %) 3-(1-Methylcyclohexen-4-yl)-butylamin, $Kp._{10}$ 120°C.

**Ungesättigtes Amin C:**

620 g Campholenaldehyd werden in einem Rührautoklaven in 600 ml flüssigem Ammoniak gelöst und bei 90°C und einem Wasserstoffdruck von 100 bar über 50 g Raney-Nickel-Eisen hydriert. Nach dem Abdampfen des Ammoniaks wird vom Katalysator abfiltriert und i. Vak. destilliert. Man erhält 500 g (80 %) 4-Aminoethyl-1,5,5-trimethyl-cyclopenten, $Kp._{10}$ 85 - 90°C.

**Beispiel 1**

a) Herstellung von 1-Amino-1-methyl-4(3)-aminomethyl-cyclohexan:
Zwischen 10 und 25°C tropft man 1125 g des ungesättigten Amins A zu 2650 g 70 % Schwefelsäure, bei 40 bis 45°C tropft man 360 ml Blausäure zu, rührt 4 Stunden bei 45°C nach, destilliert i. Vak. die nicht umgesetzte Blausäure ab, gibt 2 l Wasser hinzu, erhitzt 3 Stunden am Rückfluß, stellt mit 2,7 l 45 % Natronlauge alkalisch, dekantiert vom abgeschiedenen Salz, trennt die organische Phase ab, wäscht das Salz und die wäßrige Phase zweimal mit Toluol und destilliert über eine 80-cm-Kolonne i. Vak. Man erhält 907 g 1-Amino-1-methyl-4(3)-aminomethylcyclohexan, $Kp._{10}$ 95 - 105°C und 172 g 4(3)-Aminomethyl-1-methyl-cyclohexanol, $Kp._{10}$ 115 - 120°C.
b) Herstellung von 1-Amino-1-methyl-4(3)-aminomethyl-cyclohexan:
Zwischen 20 und 30°C tropft man 175 g 70 % Schwefelsäure zu 71,5 g 4(3)-Aminomethyl-1-methylcyclohexanol (Nebenprodukt der Diaminherstellung gemäß 1a)). Bei 40°C tropft man 27 ml Blausäure zu, rührt 4 Stunden bei 45°C nach, destilliert i. Vak. die nicht umgesetzte Blausäure ab, gibt 100 ml Wasser hinzu, erhitzt 3 Stunden am Rückfluß, stellt mit 200 ml 45 % Natronlauge alkalisch, dekantiert vom abgeschiedenen Salz, trennt die organische Phase ab, wäscht das Salz und die wäßrige Phase zweimal mit Toluol und destilliert i. Vak. Man erhält 53 g 1-Amino-1-methyl-4(3)-aminomethylcyclohexan, $Kp._{10}$ 95-105°C.
c) Herstellung von 1-Isocyanato-1-methyl-4(3)-isocyanato-methylcyclohexan (1. Phosgeniermethode):
Zu einer Lösung von 250 g Phosgen in 350 ml Dichlorbenzol tropft man bei 0°C eine Lösung von 71 g 1-Amino-1-methyl-4(3)-aminomethylcyclohexan in 150 ml Dichlorbenzol. Unter Einleiten von Phosgen erwärmt man in 2 Stunden auf 150°C und leitet bei 150°C 7 Stunden Phosgen ein. Das Phosgen wird mit Stickstoff ausgeblasen, das Lösungsmittel wird i. Vak. abdestilliert und der Rückstand in Vak. fraktioniert. Man erhält 73 g 1-Isocyanato-1-methyl-4(3)-isocyanato-methylcyclohexan, $Kp._{0,1}$ 95 - 103°C. Gewichtsverhältnis der 4- bzw. 3-Isomeren ca. 80:20.
d) Herstellung von 1-Isocyanato-1-methyl-4(3)-isocyanatomethyl-cyclohexan (2. Phosgeniermethode):
Eine Lösung von 71 g 1-Amino-1-methyl-4(3)-amino-methyl-cyclohexan in 1 l Dichlorbenzol wird bei 90°C mit $CO_2$ gesättigt und 6 Stunden bei 90°C gerührt. Bei 0°C werden 100 g Phosgen einkondensiert. Unter Durchleiten von Phosgen wird in 2 Stunden auf 150°C erwärmt. Bei 150°C leitet man 10 Stunden Phosgen ein, bläst das Restphosgen mit Stickstoff aus, destilliert das Lösungsmittel i. Vak. und fraktioniert den Rückstand i. Vak. Man erhält 71 g 1-Isocyanato-1-methyl-4(3)-isocyanatomethyl-cyclohexan, $Kp._{0,1}$ 95 - 103°C. Gewichtsverhältnis der 4- bzw. 3-Isomeren ca. 80:20.

**Beispiel 2**

a) Herstellung von 1-Amino-1-methyl-4-(4-aminobut-2-yl)-cyclohexan:
Zu 1200 g 70 % Schwefelsäure tropft man bei 5 bis 10°C 660 g des ungesättigten Amins B. Bei 30 - 35°C tropft man 170 ml Blausäure hinzu, rührt 4 Stunden bei 45°C nach, destilliert i. Vak. die überschüssige Blausäure ab, fügt 300 ml Wasser hinzu, erhitzt 3 Stunden am Rückfluß, stellt mit 1400 ml 45 % Natronlauge alkalisch, dekantiert vom abgeschiedenen Salz, trennt die Phasen, extrahiert das Salz und die wäßrige Phase zweimal mit Toluol und destilliert i. Vak. Man erhält 656 g (90 %) 1-Amino-1-methyl-4-(4-aminobut-2-yl)-cyclohexan, $Kp._{10}$ 135 - 137°C.
b) Herstellung von 1-Isocyanato-1-methyl-4-(4-isocyanatobut-2-yl)-cyclohexan:
Zu einer Lösung von 125 g Phosgen in 200 ml Dichlorbenzol tropft man bei 0°C eine Lösung von 46 g 1-Amino-1-methyl-4-[4-aminobut-2-yl] cyclohexan in 80 ml Dichlorbenzol. Unter Einleiten von Phosgen erwärmt man in 2 Stunden auf 150°C und leitet bei 150°C noch 3 Stunden Phosgen ein. Das Restphosgen wird mit Stickstoff ausgeblasen. Das Lösungsmittel wird i. Vak. destilliert und der Rückstand i. Vak. fraktioniert. Man erhält 50 g (85 %) 1-Isocyanato-1-methyl-4-(4-isocyanatobut-2-yl)-cyclohexan, $Kp._{0,1}$ 130°C.

**Beispiel 3**

a) Herstellung von 1-Amino-1,2,2-trimethyl-3-(2-aminoethyl)-cyclopentan:
Zu 80 ml Blausäure tropft man bei 0 bis 5°C 100 g 80 % Schwefelsäure und dann bei 5 bis 10°C 50 g des

ungesättigten Amins C, rührt 4 Stunden bei 45°C nach, destilliert die überschüssige Blausäure ab, fügt 80 ml Wasser hinzu, erhitzt 3 Stunden am Rückfluß, stellt mit 200 g 45 % Natronlauge alkalisch, dekantiert vom abgeschiedenen Salz, trennt die Phasen, extrahiert das Salz und die wäßrige Phase zweimal mit Toluol und destilliert i. Vak. Man erhält 40 g (72 %) 1-Amino-1,2,2-trimethyl-3-(2-aminoethyl)-cyclopentan, $Kp._{10}$ 125°C.

b) Herstellung von 1-Isocyanato-1,2,2-trimethyl-3-(2-isocyanatoethyl)-cyclopentan:

Zu einer Lösung von 125 g Phosgen in 200 ml Dichlorbenzol tropft man bei 0°C eine Lösung von 42,5 g 1-Amino-1,2,2-trimethyl-3-[2-aminoethyl]-cyclopentan in 80 ml Dichlorbenzol. Unter Durchleiten von Phosgen erwärmt man in 2 Stunden auf 150°C und leitet bei 150°C noch 4 Stunden Phosgen ein. Das Restphosgen wird mit Stickstoff ausgeblasen. Das Lösungsmittel wird i. Vak. abdestilliert und der Rückstand i. Vak. fraktioniert. Man erhält 33 g (60 %) 1-Isocyanato-1,2,2-trimethyl-3-(2-isocyanatoethyl)-cyclopentan, $Kp._{0,1}$ 120°C.

**Patentansprüche**

1. Gegebenenfalls als Isomerengemische vorliegende Diisocyanate der Formel I

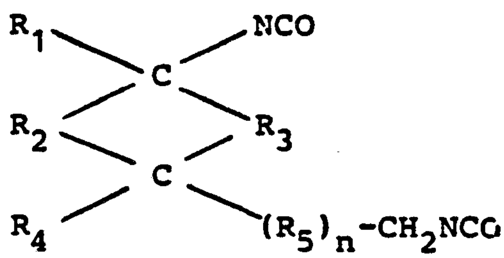

I

in welcher

$R_1$ für einen Alkylrest mit 1 bis 4 Kohlenstoffatomen steht,

$R_2$ und $R_3$ für gleiche oder verschiedene Reste stehen und jeweils lineare oder verzweigte, zweiwertige Kohlenwasserstoffreste mit 1 bis 4 Kohlenstoffatomen bedeuten,

$R_4$ für Wasserstoff oder einen Alkylrest bit 1 bis 4 Kohlenstoffatomen steht,

$R_5$ für einen linearen oder verzweigten, gesättigten zweiwertigen Kohlenwasserstoffrest mit 1 bis 4 Kohlenstoffatomen steht und

n für 0 oder 1 steht.

2. Gegebenenfalls Isomerengemische darstellende Diisocyanate der in Anspruch 1 genannten Formel I, dadurch gekennzeichnet, daß

$R_1$ für einen Methylrest steht,

$R_2$ und $R_3$ gleiche oder verschiedene lineare, zweiwertige, gesättigte Kohlenwasserstoffreste mit 1 bis 3 Kohlenstoffatomen bedeuten, mit der Maßgabe, daß die Summe der Anzahl der Kohlenstoffatome der Reste $R_2$ und $R_3$ 3 oder 4 beträgt,

$R_4$ für Wasserstoff oder einen Methylrest steht und

$R_5$ und n die in Anspruch 1 genannte Bedeutung haben.

3. 1-Isocyanato-1-methyl-4(3)isocyanatomethyl-cyclohexan.

4. 1-Isocyanato-1-methyl-4-(4-isocyanatobut-2-yl)-cyclohexan.

5 1-Isocyanato-1,2,2-trimethyl-3-(2-isocyanatoethyl)-cyclopentan.

6. Verwendung der, gegebenenfalls als Isomerengemisch vorliegenden Diisocyanate gemäß Anspruch 1 bis 5, gegebenenfalls in mit Blockierungsmitteln für Isocyanatgruppen blockierter Form als Isocyanatkomponente bei der Herstellung von Polyurethankunststoffen.

**Claims**

1. Diisocyanates, optionally present in the form of isomer mixtures, of the formula

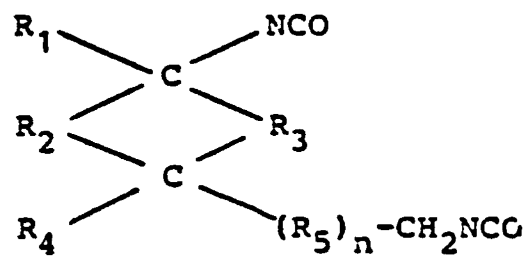

I

in which

$R_1$ reperesents an alkyl radical with 1 to 4 carbon atoms,

$R_2$ and $R_3$ represent identical or different radicals and each denote linear or branched, divalent hydrocarbon radicals with 1 to 4 carbon atoms,

$R_4$ represents hydrogen or an alkyl radical with 1 to 4 carbon atoms,

$R_5$ represents a linear or branched, saturated divalent hydrocarbon radical with 1 to 4 carbon atoms and

n represents 0 or 1.

2. Diisocyanates, optionally representing isomer mixtures, of the formula I mentioned in Claim 1, characterised in that

$R_1$ represents a methyl radical,

$R_2$ and $R_3$ denote identical or different linear, divalent, saturated hydrocarbon radicals with 1 to 3 carbon atoms, with the proviso that the sum of the number of carbon atoms of the radicals $R_2$ and $R_3$ is 3 or 4,

$R_4$ represents hydrogen or a methyl radical and

$R_5$ and n have the meaning mentioned in Claim 1.

3. 1-Isocyanato-1-methyl-4(3)iscyanatomethyl-cyclohexane.

4. 1-Isocyanato-1-methyl-4-(4-isocyanatobul-2-yl)-cyclohexane.

5. Isocyanato-1,2,2-trimethyl-3-(2-isocyanatoethyl)-cyclopentane.

6. Use of the diisocyanates, optionally present in the form of an isomer mixture, according to Claim 1 to 5, optionally in a form blocked with blocking agents for isocyanate groups, as the isocyanate component in the production of polyurethane plastics.

**Revendications**

1. Diisocyanates existant éventuellenent sous forme de mélanges d'isomères, de formule I

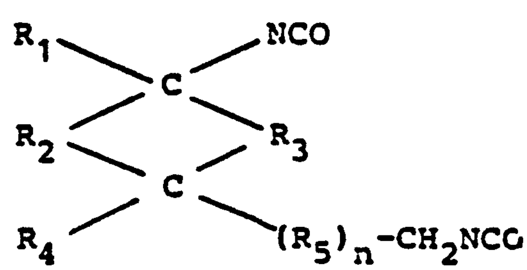

I

dans laquelle

$R_1$ représente un reste alkyle ayant 1 à 4 atomes de carbone,

$R_2$ et $R_3$ représentent des restes identiques ou différents et désignent chacun des restes hydrocarbonés divalents linéaires ou ramifiés ayant 1 à 4 atomes de carbone,

$R_4$ représente l'hydrogène ou un reste alkyle ayant 1 à 4 atomes de carbone,

$R_5$ désigne un reste hydrocarboné divalent saturé, linéaire ou ramifié, ayant 1 à 4 atomes de carbone et

$\underline{n}$ a la valeur 0 ou 1.

2. Diisocyanates représentant éventuellement des mélanges d'isomères, de formule I suivant la revendication 1, caractérisés en ce que

$R_1$ est un reste méthyle,

$R_2$ et $R_3$ sont des restes hydrocarbonés saturés divalents linéaires, identiques ou différents, ayant 1 à 3 atomes de carbone, sous réserve que la somme des nombres d'atomes de carbone des restes $R_2$ et $R_3$ ait une valeur de 3 ou 4,

$R_4$ représente l'hydrogène ou un reste méthyle et

$R_5$ et $\underline{n}$ ont la définition indiquée dans la revendication 1.

3. Le 1-isocyanato-1-méthyl-4(3)isocyanato-méthylcyclohexane.

4. Le 1-isocyanato-1-méthyl-4-(4-isocyanatobut-2-yl)cyclohexane.

5. Le 1-isocyanato-1,2,2-triméthyl-3-(2-isocyanatoéthyl)cyclopentane.

6. Utilisation des diisocyanates se présentant éventuellement sous forme de mélanges d'isomères suivant les revendications 1 à 5, le cas échéant sous une forme protégée avec des agents de protection pour groupes isocyanato, comme composant isocyanate dans la production de matières plastiques du type polyuréthanne.